# EUROPEAN PATENT APPLICATION

(11) **EP 4 613 226 A1**
(43) Date of publication of application: **10.09.2025**
(21) Application number: 23884044.1
(22) Date of filing: 24.03.2023
(51) Int. Cl.: A61B 34/30

(54) **FLEXIBLE SURGICAL INSTRUMENT, AND FLEXIBLE INSTRUMENT AND INSTRUMENT DELIVERY UNIT THEREOF**

(30) Priority: 04.11.2022 CN 202211373251
(71) Applicant: Beijing Yunlijing'an Technology CO., Ltd, Beijing 102600 (CN)
(72) Inventor: JIANG, Wei, Beijing 102600 (CN); WU, Wenjie, Beijing 102600 (CN); LU, Tianwei, Beijing 102600 (CN)
(74) Representative: Grünecker Patent- und Rechtsanwälte PartG mbB
(86) International application number: PCT/CN2023/083621
(87) International publication number: WO 2024/093098

(57) **Abstract**

An instrument delivery unit (11), comprising a housing (111) and an instrument storage device (112), wherein the housing (111) has an internal accommodation space and is provided with an instrument outlet (1113) on a side wall; the instrument storage device (112) is provided with a spiral accommodation groove (11211) on a peripheral surface; the housing (111) comprises a limiting guide portion (1112) and a first transmission thread (1111), and on the basis of the configuration of the limiting guide portion (1112), the housing (111) can axially move and is restricted from circumferential rotation; and correspondingly, the instrument storage device (112) is provided with a second transmission thread (11212) that is in transmission connection with the first transmission thread (1111), and on the basis of a transmission connection relationship, the instrument storage device (112) rotates to drive the housing (111) to axially move. Also provided are a flexible instrument comprising the instrument delivery unit (11), and a flexible surgical instrument. The present application can achieve the continuous delivery of a flexible body of an effector unit, and has a simple structure and low maintenance costs.

## Description

This application claims the priority of the Chinese Patent Application No. 202211373251.5, titled "FLEXIBLE SURGICAL INSTRUMENT, AND FLEXIBLE INSTRUMENT AND INSTRUMENT DELIVERY UNIT THEREOF", filed on November 04, 2022 with the China National Intellectual Property Administration, which is incorporated herein by reference in its entirety.

### FIELD

The present application relates to the technical field of medical instruments and, in particular, to a flexible surgical instrument, a flexible instrument and an instrument delivery unit thereof.

### BACKGROUND

In recent years, natural orifice diseases such as digestive, urinary and respiratory diseases have seriously endangered human health, and incidence and mortality of diseases such as gastric cancer, esophageal cancer, bladder cancer and lung cancer have been increasing year by year. The diagnosis and treatment performed with flexible endoscopes combined with related surgical instruments have the advantages of being minimally invasive, of rapid postoperative recovery, of reducing medical costs and the like, and have become a mainstream treatment for such diseases.

In addition, compared with the conventional large-incision surgery, a surgery performed through the natural orifice of human body has a small operation space and is limited, and generally relies on flexible instruments for diagnosis and treatment. There are many kinds of conventional flexible instruments, such as but not limited to clamp instruments, electrosurgical coagulation and cutting instruments, injection instruments, and guide instruments, which can meet different operating requirements in narrow environments. At present, endoscopic surgical instruments are designed based on manual operation, and requires manual operations of professionally trained technicians or nurses and other medical personnel. Without technicians or nurses, it is difficult for a doctor to complete diagnosis and treatment alone. In order to meet the requirements of diagnosis and treatment of natural orifice, the conventional flexible instruments are designed as flexible and slender instruments, which require manual operation and close cooperation of doctors and nurses during the use of the flexible instruments, and the operation of the flexible instruments is complicated. Moreover, the flexible and slender instruments are easily contaminated due to contact with foreign matters such as body fluids, and there is also a risk of cross-infection during the retrieval of the instruments. In order to solve the above problems, an endoscopic nurse/technician robot has been developed to assist endoscopists in operating the surgical instruments.

In view of above, it is desired to optimize the design of flexible instruments to overcome the above defects.

### SUMMARY

An object of the present application is to provide a flexible surgical instrument, a flexible instrument and an instrument delivery unit thereof, which can effectively accommodate a body of an actuator unit of the flexible instrument by optimized configuration, meets the functional requirements of avoiding cross-contamination, and has a reasonable and reliable structural design.

An instrument delivery unit according to an embodiment of the present application, for delivering and storing an actuator unit having a flexible body, includes a housing and an instrument storage device. The housing has an internal accommodating space and an instrument outlet is provided in a side wall of the housing. A first transmission thread is provided on the housing, and a limiting guide portion is provided outside the housing. The limiting guide portion is configured to be fitted with a fixed member or structure and is configured to guide an axial movement of the housing and prevent a circumferential rotation of the housing. At least part of the instrument storage device is arranged in the housing, and an outer circumferential surface of the instrument storage device is provided with a helical accommodating groove for winding and storing the flexible body of the actuator unit. The instrument storage device is provided with a second transmission thread which is in transmission connection with the first transmission thread.

Optionally, the first transmission thread is arranged on an inner wall surface of the housing, and the outer circumferential surface of the instrument storage device is provided with a helical groove which includes an inner groove section and an outer groove section formed in sequence in a radial direction. A wall of the inner groove section defines the helical accommodating groove, and a wall of the outer groove section forms the second transmission thread.

Optionally, a lead screw is arranged in the middle of the housing, and the first transmission thread is arranged on the lead screw. A nut is fixedly provided in the instrument storage device, and the nut is provided with the second transmission thread.

Optionally, the housing is cylindrical and has one open end. One end of the instrument storage device is arranged in the housing, and the other end of the instrument storage device is provided with a delivery drive connector.

A flexible instrument is also provided according to the present application, which includes the instrument delivery unit described above, an actuator unit and a transmission unit. The actuator unit includes an actuator and a flexible body. The flexible body includes a drive wire and a sleeve in which the drive wire is arranged, and the actuator is arranged at a distal end of the drive wire. The transmission unit is connected to the instrument storage device of the instrument delivery unit, and is configured to drive the actuator to move via the drive wire of the actuator unit. The transmission unit is provided with an execution drive connector for being fitted with an instrument driving device. The transmission unit includes a transmission base plate fixedly connected to the instrument storage device, and a push-pull transmission assembly and a rotation transmission assembly that are arranged on the transmission base plate. The push-pull transmission assembly is configured to push out or retract the drive wire, and the rotation transmission assembly is configured to twist the drive wire.

Optionally, the push-pull transmission assembly includes a fixing disc and a first driving shaft. The first driving shaft is inserted into and arranged on the transmission base plate and is configured to be in transmission connection with the instrument driving device via the execution drive connector. The fixing disc is coaxially and fixedly connected to the first driving shaft, and a proximal end of the drive wire is connected to the fixing disc. The drive wire is configured to be pushed out or retracted along a predetermined trajectory under the driving of the fixing disc, and the drive wire has a rotational degree of freedom relative to the fixing disc.

Optionally, a first terminal is embedded in an outer circumferential surface of the fixing disc, and the first terminal has a first through hole. The drive wire passes through the first through hole of the first terminal, and a body of the drive wire is provided with two limit bumps which are respectively located at two ends of the first through hole of the first terminal. A radial gap is provided between the drive wire and the first terminal at the first through hole, and a size of each of the two limit bumps is larger than that of the first through hole.

Optionally, a rotation limiting pair is provided between the fixing disc and the transmission base plate, and the rotation limiting pair is configured to limit a rotation angle of the fixing disc.

Optionally, a body of the fixing disc is provided with an arc groove, and a circle center of the arc groove is concentric with a rotation center of the fixing disc. The transmission base plate is provided with a limit block, and the limit block is arranged in the arc groove. Two opposite ends of the arc groove are configured to abut against the limit block to limit the rotation angle of the fixing disc.

Optionally, a first constraint member is fixedly arranged on the transmission base plate, and the first constraint member includes a cylindrical guide body and a holding body, and the fixing disc is arranged in the guide body. Each of the guide body and the holding body is provided with a constraint cavity for accommodating the drive wire, so that the drive wire is pushed out or retracted along the predetermined trajectory. The constraint cavity on an inner wall of the guide body is an open cavity, and the constraint cavity of the holding body is a closed cavity.

Optionally, the rotation transmission assembly includes a rotation shaft, a second terminal, a bevel gear set and a second driving shaft. The second driving shaft is inserted into and arranged on the transmission base plate, and is configured to be in transmission connection with the instrument driving device via the execution drive connector. A driving gear of the bevel gear set is connected to the second driving shaft, and the rotation shaft is connected to a driven wheel of the bevel gear set. The drive wire is fixed to the second terminal. The second terminal is arranged on the rotation shaft and is configured to rotate under the driving of the rotation shaft. The second terminal has a degree of freedom of sliding in the pulling direction of the drive wire relative to the rotation shaft.

Optionally, the second terminal is embedded in a mounting hole in the middle of the rotation shaft, and the second terminal and the mounting hole have rectangular cross sections matched with each other, and the drive wire is configured to extend into the constraint cavities of the first constraint member through the mounting hole.

Optionally, a second constraint member is fixedly provided on the transmission base plate. One end of the second constraint member faces one end of the holding section of the first constraint member in an axial direction of the rotation shaft, and two opposite ends of the rotation shaft are respectively pivoted with the second constraint member and the first constraint member.

Optionally, the second constraint member is provided with a second through hole, and a size of the second through hole of the second constraint member is adapted to a size of the sleeve of the actuator unit to fix an end of the sleeve. The instrument storage device is provided with a pass-through port, and the flexible body of the actuator unit extends and transitions into a helical accommodating groove on an outer surface of the instrument storage device through the pass-through port of the instrument storage device.

A flexible surgical instrument is also provided according to the present application, which includes the flexible instrument described above and an instrument driving device for outputting a driving force to the flexible instrument.

Optionally, the instrument driving device includes: a first driving part including an output end for outputting a rotational driving force; a casing in transmission connection with the output end of the first driving part; a driving base plate fixedly arranged on the casing and configured to transmit the rotational driving force to the flexible instrument; a second driving part and a third driving part fixedly arranged on the casing by a mounting base plate. Each of the second driving part and the third driving part includes an output end for outputting the rotational driving force, and the output ends are configured to transmit the rotational driving forces to the push-pull transmission assembly and the rotation transmission assembly of the transmission unit, respectively.

Optionally, the instrument driving device further includes a first driving transmission disc and a second driving transmission disc. The first driving transmission disc is in transmission connection with the output end of the second driving part, and the second driving transmission disc is in transmission connection with the output end of the third driving part. The transmission unit further includes a first driven transmission disc connected to the push-pull transmission assembly and a second driven transmission disc connected to the rotation transmission assembly. The first driving transmission disc is in transmission connection with the first driven transmission disc, and the second driving transmission disc is in transmission connection with the second driven transmission disc.

Optionally, one of the first driving transmission disc and the first driven transmission disc is provided with a connecting hole extending in an axial direction, and the other of the first driving transmission disc and the first driven transmission disc is provided with a connecting post extending in the axial direction, and the connecting hole and the connecting post that are matched with each other are arranged eccentrically with respect to rotation centers of the first driving transmission disc and the first driven transmission disc. One of the second driving transmission disc and the second driven transmission disc is provided with a connecting hole extending in the axial direction, and the other of the second driving transmission disc and the second driven transmission disc is provided with a connecting post extending in the axial direction, and the connecting hole and the connecting post that are matched with each other are arranged eccentrically with respect to rotation centers of the second driving transmission disc and the second driven transmission disc.

Optionally, the driving base plate is provided with a first step hole and a second step hole, and each of the first step hole and the second step hole includes a small-diameter section and a large-diameter section which are sequentially arranged from top to bottom to form a step. The first driving transmission disc is rotatably arranged in the large-diameter section of the first step hole, and the second driving transmission disc is rotatably arranged in the large-diameter section of the second step hole.

Optionally, a surface of each of the first driving transmission disc and the second driving transmission disc facing the corresponding driving part is provided with a key groove. The output end of each of the second driving part and the third driving part is provided with a sliding shaft sleeve, and one end of the sliding shaft sleeve facing the corresponding driving transmission disc is provided with an outer sliding key. The outer sliding key is inserted into the corresponding key groove, and the outer sliding key and the corresponding key groove are configured to rotate coaxially and slide axially relative to each other. A transmission disc return spring is provided between each of the first driving transmission disc and the second drive disc, and the corresponding sliding shaft sleeve. The transmission disc return spring is configured to be pre-compressed between the sliding shaft sleeve and the corresponding driving transmission disc under a normal condition.

Optionally, the mounting base plate is provided with a detection element, and the detection element is located on an axial movement trajectory of the first driving transmission disc and the second driving transmission disc.

Compared with the conventional technology, there is proposed in the present application a storage and delivery implementation scheme for the actuator unit with the flexible body. Specifically, the housing of the instrument delivery unit has an internal accommodating space, and the instrument outlet on the side wall of the instrument delivery unit. The outer peripheral surface of the instrument storage device unit is provided with the helical accommodating groove for winding and storing the flexible body of the actuator unit. The housing includes the limiting guide portion and the first transmission thread. By providing the limiting guide portion, the housing can move axially and restricted from rotating in the circumferential direction. Correspondingly, the instrument storage device is provided with the second transmission thread in transmission connection with the first transmission thread. With this transmission connection, the rotation of the instrument storage device can drive the housing to move axially. The application of this solution has the following beneficial technical effects.

Firstly, as the instrument storage device rotates, the flexible body wound and stored in the helical accommodating groove can be continuously conveyed through the instrument outlet. At the same time, the instrument storage device drives the housing to move axially synchronously, so that the flexible body separated from the helical accommodating groove can maintain a certain relative position relationship with the instrument outlet of the housing. During the delivery of the actuator, the elastic deformation energy stored due to the spiral winding deformation can be released, which can effectively overcome the resistance generated by entering a flexible endoscope and assist in providing good delivery. Moreover, this solution is suitable for an Endoscopic Nurse/Technician Robot, which can effectively improve the work efficiency of doctors.

Secondly, compared with the method of providing the driving forces in both rotation and axial dimensions at a driving side, this solution has the characteristics of simple structure based on the rotational driving force for driving the instrument storage device and the axial movement of the housing on an instrument side to adapt to the position where the flexible body is disengaged from the helical accommodating groove during the delivery. Moreover, this arrangement can also reduce manufacturing cost and maintenance cost of the driving mechanism.

Thirdly, in an optional solution of the present application, the first constraint member includes the cylindrical guide body and the holding body, and the fixing disc is configured to push out or retract the drive wire along the predetermined trajectory. The fixing disc is arranged in the cylindrical guide body. The actuation performance of the fixing disc can be further ensured under the rotating relationship, and the structure is reliable.

Fourthly, in another optional solution of the present application, the rotation limit pair is arranged between the fixing disc and the transmission base plate, and the rotation limit pair is configured to limit the rotation angle of the fixing disc. Further, the body of the fixing disc is provided with the arc groove, and the circle center of the arc groove is concentric with the rotation center of the fixing disc. Accordingly, the limit block is arranged on the transmission base plate and is arranged in the arc groove, and the two side ends of the arc groove are configured to abut against the limit block to limit the rotation angle of the fixing disc. In this way, the bidirectional limit positions of the stretching movement can be located, that is, the limit working positions of the push-pull transmission assembly can be constructed based on the arc groove and the limit block, which can avoid the excessive rotation angle of the fixing disc to cause the damage to the drive wire and affect the actuation performance, while meeting the requirements of a pulling stroke of the first terminal.

Fifthly, in another optional solution of the present application, the driving base plate is provided with two step holes, and each of the two step holes includes the small-diameter section and the large-diameter section which are sequentially arranged from top to bottom to form the step. The first driving transmission disc and the second driving transmission disc are rotatably arranged in the large-diameter sections of the two step holes, respectively. Moreover, the matched pair that can rotate coaxially and slide axially relative to each other is constructed between each of the first driving transmission disc and the second driving transmission disc, and the corresponding driving part. The first driving transmission disc and the second driving transmission disc are each provided with the transmission disc return springs. The transmission disc return springs are pre-compressed and pressed against the corresponding driving transmission discs under the normal condition. During the actual installation operation, the driven transmission discs are respectively aligned with the corresponding driving transmission discs and pressed for mounting. Based on the axial relative sliding matched pair between the two, during the pressing process of an operator, the driven transmission disc can press the corresponding driving transmission disc to move downwards synchronously, and the transmission disc return spring is pressed and deformed to provide buffering adaptability, which can avoid the rigid interference influence during the insertion process. After connected in place, the transmission disc return spring releases elastic deformation energy and pushes the driven transmission disc to move axially to abut against the step of the corresponding step hole.

In addition, according to the present application, the detection element is further provided and is located on the axial movement trajectory of the first driving transmission disc and the second driving transmission disc. When the flexible instrument is installed on the driving device, the two driving transmission discs are pressed to move downwards synchronously, and then press the pressure-sensitive detection element to generate a signal. When the connection is done, the pressure-sensitive detection element loses pressure and the signal disappears. Thus, the installation state is detected. In this way, the operator can realize blind connection between the instrument side and the driving side, which can further improve the operability.

### BRIEF DESCRIPTION OF THE DRAWINGS

FIG. 1 is a schematic view of a flexible surgical instrument in use according to an embodiment;
FIG. 2 is a top view of the flexible surgical instrument shown in FIG. 1;
FIG. 3 is a schematic view of an overall structure of a flexible instrument according to an embodiment;
FIG. 4 is a top view of FIG. 3;
FIG. 5 is a cross-sectional view taken along line A-A in FIG. 4;
FIG. 6 is an enlarged view of part B in FIG. 5;
FIG. 7 is an exploded view of an assembly of a flexible instrument according to an embodiment;
FIG. 8 is a schematic view showing a flexible instrument in use according to an embodiment;
FIG. 9 is a schematic view of an overall structure of a transmission unit according to an embodiment;
FIG. 10 is a cross-sectional view taken along line C-C in FIG. 8;
FIG. 11 is an enlarged view of part D in FIG. 10;
FIG. 12 is an enlarged view of part G in FIG. 10;
FIG. 13 is a schematic view showing assembly of a connecting unit according to an embodiment;
FIG. 14 is a schematic view showing assembly of a two-way connector according to an embodiment;
FIG. 15 is a top view of an instrument driving device according to an embodiment;
FIG. 16 is a cross-sectional view taken along line H-H in FIG. 15;
FIG. 17 is a partial sectional view taken along line I-I in FIG. 15, showing assembly between a driving base plate and a snap fastener;
FIG. 18 is an enlarged view of part J of FIG. 16;
FIG. 19 is a schematic view showing assembly of a flexible instrument according to another embodiment;
FIG. 20 is an axial cross-sectional view of the flexible instrument shown in FIG. 19; and
FIG. 21 is a schematic view showing an application scenario of a flexible surgical instrument according to an embodiment.

**List of reference signs in the figures:**

| | | | | | |
|---|---|---|---|---|---|
| flexible instrument | 10, | instrument delivery unit | 11, | housing | 111, |
| first transmission thread | 1111, | limiting guide portion | 1112, | instrument outlet | 1113, |
| instrument storage device | 112, | helical groove | 1121, | helical accommodating groove | 11211, |
| second transmission thread | 11212, | first mounting seat | 1122, | | |
| pass-through port | 1123, | engagement opening | 1124, | protective tube | 113, |
| actuator unit | 12, | drive wire | 121, | limit bump | 1211, |
| actuator | 122, | sleeve | 123, | transmission unit | 13, |
| push-pull transmission assembly | 131, | first driving shaft | 1311, | | |
| first terminal | 1312, | first through hole | 13121, | fixing disc | 1313, |
| mounting groove | 13131, | arc groove | 13132, | limit block | 1314, |
| first constraint member | 1315, | | | constraint cavity | 13151, |
| rotation transmission assembly | 132, | | | rotation shaft | 1321, |
| mounting hole | 13211, | second terminal | 1322, | spur gear set | 1323, |
| driving gear | 13231, | driven gear | 13232, | bevel gear set | 1324, |
| driving bevel gear | 13241, | driven bevel gear | 13242, | second driving shaft | 1325, |
| second constraint member | 1326 | | | second through hole | 13261, |
| transmission base plate | 133, | engagement groove | 1331, | first mounting hole | 1332, |
| first driven transmission disc | 134, | connecting hole | 1341, | guide hole | 1342, |
| second driven transmission disc | 135, | connecting unit | 14, | two-way connector | 141; |
| instrument driving device | 20, | first driving part | 21, | second driving part | 22, |
| third driving part | 23, | connecting part | 24, | driving base plate | 241, |
| first step hole | 2411, | second step hole | 2412, | first driving transmission disc | 242, |
| key groove | 2421, | connecting post | 2422, | guide post | 2423, |
| second driving transmission dis | 243, | snap fastener | 244, | hook portion | 2441, |
| button | 245, | snap fastener return spring | 246, | casing | 247, |
| pass-through hole | 2471, | fixing support | 25, | limiting slide groove | 251, |
| conveying gear set | 26, | input gear | 261, | output gear | 262, |
| sliding shaft sleeve | 27, | | | outer sliding key | 271, |
| transmission disc return spring | 28, | | | detection element | 29; |
| housing | 111a, | first transmission thread | 1111a, | lead screw | 1114a, |
| instrument storage device | 112a, | | | helical commodating groove | 11211a, |
| second transmission thread | 11212a, | | | nut | 1125a; |
| flexible surgical instrument | 100, | | | robot body | 200. |

### DETAILED DESCRIPTION OF THE EMBODIMENTS

Hereinafter, in order to enable those skilled in the art to better understand the technical solution of the present application, the present application will be further described in detail with reference to the accompanying drawings and embodiments.

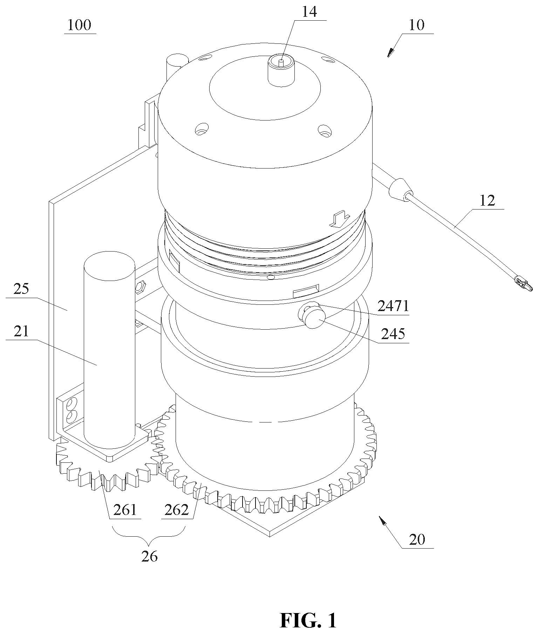

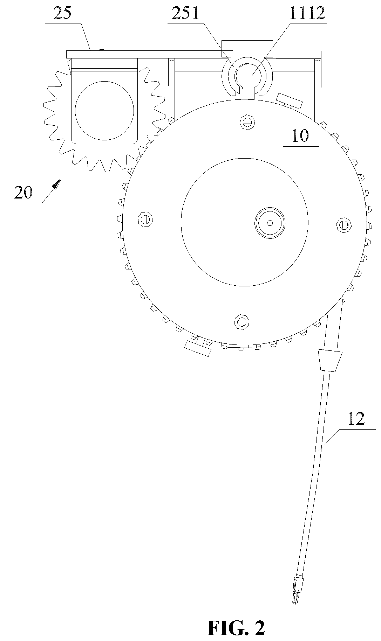

Without losing generality, a flexible surgical instrument is provided according to an embodiment to effectively solve the problems of complicated operation, difficulty in storage, and ease of contamination of flexible and slender instruments. Referring to FIGS. 1 and 2, FIG. 1 is a schematic view of the overall structure of the flexible surgical instrument according to the embodiment, and FIG. 2 is a top view of the flexible surgical instrument shown in FIG. 1.

The flexible surgical instrument 100 comprises a flexible instrument 10 and an instrument driving device 20. An actuator unit 12 for diagnosis and treatment and assistance in diagnosis and treatment is provided in the flexible instrument 10, and the instrument driving device 20 can provide a driving force to the flexible instrument 10 to realize the conveying operation of the flexible instrument and rotation or opening or closing operation of an actuator.

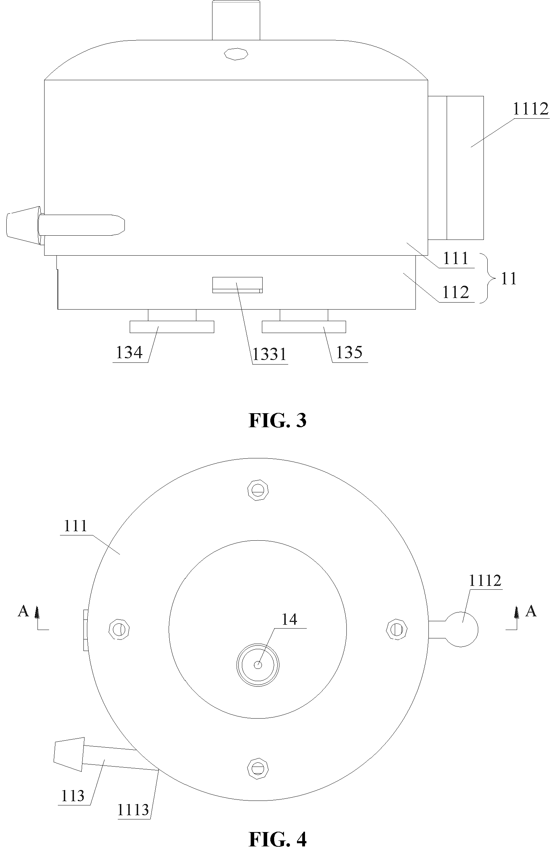

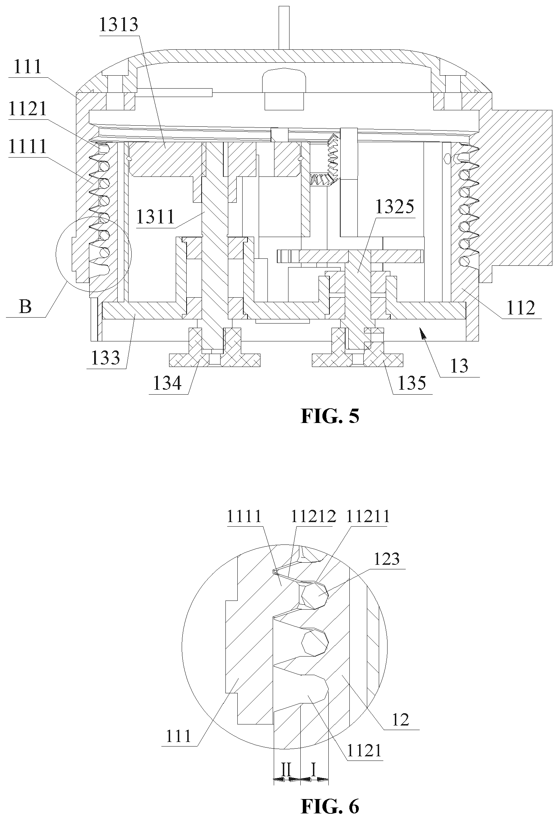

Referring to FIGS. 3, 4 and 5 together, FIG. 3 is a schematic view of an overall structure of the flexible instrument according to the embodiment; FIG. 4 is a top view of FIG. 3; and FIG. 5 is a cross-sectional view taken along line A-A in FIG. 4.

The flexible instrument 10 includes an instrument delivery unit 11, the actuator unit 12 built into the instrument delivery unit 11, and a transmission unit 13 for transmitting a driving force of the movement of the actuator.

The instrument delivery unit 11 includes a housing 111 and an instrument storage device 112. The instrument storage device 112 can be rotated relative to the housing 111 under the drive of the transmission unit 13. In the embodiment, the housing 111 is cylindrical and open at one end, and part of the instrument storage device 112 is arranged in the housing 111. In other implementations, the entire instrument storage device may be arranged in the housing (not shown).

A first transmission thread 1111 is provided on the inner wall surface of the housing 111. A limiting guide portion 1112 is arranged outside the housing 111 and is fitted in a limiting slide groove 251 of the instrument driving device 20, referring to FIG. 2. The limiting slide groove 251 has a side wall extending in an axial direction, so that the limiting guide portion 1112 can be inserted into the limiting slide groove 251 and can move axially along the limiting slide groove 251. The limiting slide groove 251 is of a relatively fixed structure, which can guide the axial movement of the housing 111 and prevent the circumferential rotation of the housing 111.

In an implementation, the limiting guide portion 1112 has the same cross-sectional shape as the limiting slide groove 251, and is in clearance fit with the limiting slide groove 251, so as to obtain a good guide effect on movement. It can be understood that the actual arrangement or position of the limiting slide groove 251 can be determined based on the overall design requirements of a product, as long as the limiting slide groove 251 can be fixed relative to the housing to perform a reliable guide and limit function. The limiting slide groove 251 is not limited to the assembly relationship shown in the figures.

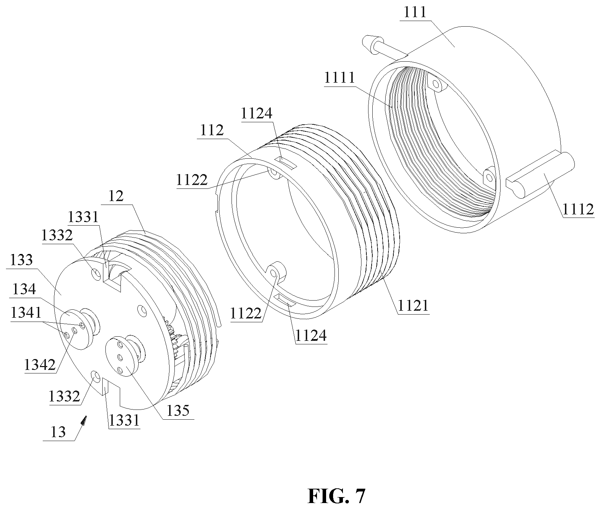

A helical groove 1121 is provided on the outer circumferential surface of the instrument storage device 112. The helical groove 1121 includes an inner groove section I and an outer groove section II which are formed in sequence in a radial direction. As shown in FIGS. 5, 6 and 7, FIG. 6 is an enlarged view of part B in FIG. 5, and FIG. 7 is an exploded view of an assembly of the flexible instrument according to the embodiment.

A wall of the inner groove section I defines a helical accommodating groove 11211 for winding and accommodating a flexible body (such as but not limited to a drive wire 121 and a sleeve 123 hereinafter) of the actuator unit 12, and the flexible body may be extended or retracted through an instrument outlet 1113 formed in a side wall of the housing 111.

A wall of the outer groove section II forms a second transmission thread 11212 which is in transmission connection with the first transmission thread 1111.

As shown in FIG. 7, the entire instrument storage device 112 is cylindrical, and the transmission unit 13 is arranged in the instrument storage device 112. In the embodiment, multiple first mounting seats 1122 are provided on an inner wall of the instrument storage device 112. Each of the first mounting seats 1122 may be provided with a threaded hole. The first mounting seat 1122 serves as a delivery drive connector. Accordingly, a transmission base plate 133 of the transmission unit 13 is provided with multiple first mounting holes 1332. The multiple first mounting seats 1122 circumferentially spaced apart from each other correspond to the multiple first mounting holes 1332 circumferentially spaced apart from each other, so as to fixedly connect the instrument storage device 112 to the transmission base plate 133 by threaded fasteners (not shown).

It can be understood that the fixed connection between the instrument storage device 112 and the transmission base plate 133 may also be realized in any other way, such as but not limited to welding, bonding or riveting.

After the instrument storage device 112 is assembled with the transmission unit 13, the instrument driving device 20 drives the transmission unit 13 to rotate and to drive the instrument storage device 112 to rotate. Based on the limiting and guiding function of the limiting guide portion 1112 and the limiting slide groove 251, the housing 111 synchronously moves axially, so that the part of the flexible body of the actuator unit 12 separated from the helical accommodating groove 11211 can be substantially aligned with the instrument outlet 1113 in two dimensions, thereby smoothly performing the retracting or extending operation.

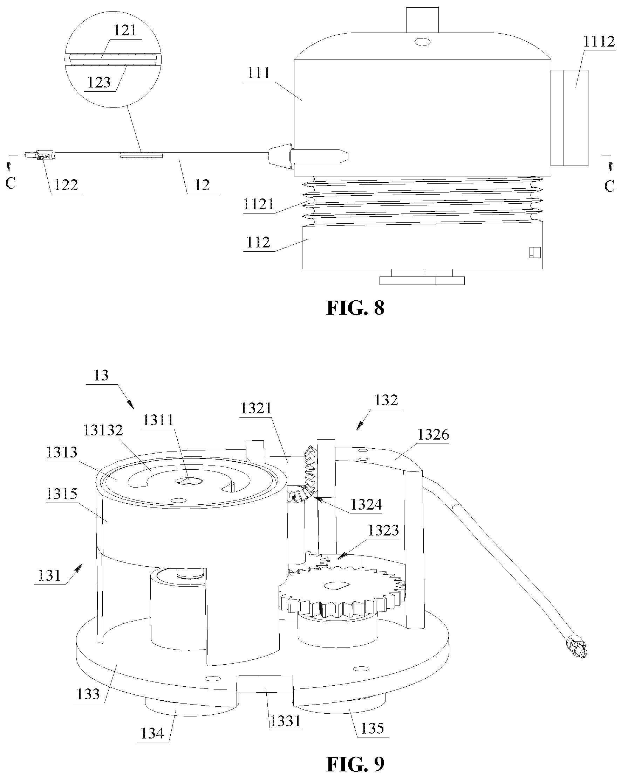

In an implementation, the circumferential and axial positions can be accurately aligned by the structural sizes of the associated components. Moreover, a protective tube 113 is provided on the outer side of the instrument outlet 1113 and is fixed to the housing 111, so that the part of the actuator unit 12 extending out of the housing 111 may be maintained stably. Referring to FIG. 8, it is a schematic view showing the flexible instrument in use according to the embodiment.

During the delivery of the actuator, the elastic deformation energy caused by the helical winding deformation can be released, which can effectively overcome a resistance generated by entering a flexible endoscope and assist in providing good delivery. As the instrument storage device 112 rotates, the flexible body of the actuator unit 12 is continuously delivered through the instrument outlet 1113. Similarly, when the instrument storage device 112 moves in the reverse direction, the flexible body can be retracted into the housing 111 and wound around the instrument storage device 112, thus realizing the retraction and storage of the actuator unit 12.

In an implementation, the actuator unit 12 may be selected according to the application scenarios, such as but not limited to clamp flexible instruments, electrosurgical coagulation and cutting flexible instruments, basket flexible instruments, injection flexible instruments, guidance flexible instruments and sensor flexible instruments, and other flexible instruments. A clamp flexible instrument includes a tissue forceps with a degree of freedom of clamping and a hemostat with a degree of freedom of rotation. An electrosurgical coagulation and cutting flexible instrument includes a clamping degree of freedom for electrosurgical coagulation and cutting of tissue and a degree of freedom for pushing and pulling snare instrument. A basket flexible instrument includes a pushing degree of freedom for pushing and retracting a basket. An injection flexible instrument includes a pushing degree of freedom for pushing and retracting a needle. A guidance flexible instrument is used to guide a coaxial instrument, and has no degree of freedom. A sensor flexible instrument may include an image sensor instrument, a position sensor instrument, a shape sensor instrument or the like.

The above functional requirements of the actuator unit 12 can be met by pulling or twisting a proximal end of the drive wire 121 of the actuator unit 12. Specifically, as shown in FIG. 8, the actuator 122 located at a distal end can be moved by pulling the drive wire 121, for example, including but not limited to, opening and closing movement of the actuator and the pushing movement of the actuator. Similarly, the rotary movement of the actuator 122 at the distal end can be realized by twisting the drive wire 121.

The directional terms "proximal end" and "distal end" used herein are defined based on the perspective of an operator of the surgical instrument, that is, one end of the drive wire 121 close to the operator is the "proximal end", and correspondingly, the other end close to a patient is the "distal end". It should be understood that the directional terms is only used to clearly describe the technical solution, and does not constitute a substantial limitation on the flexible surgical instrument according to the present application.

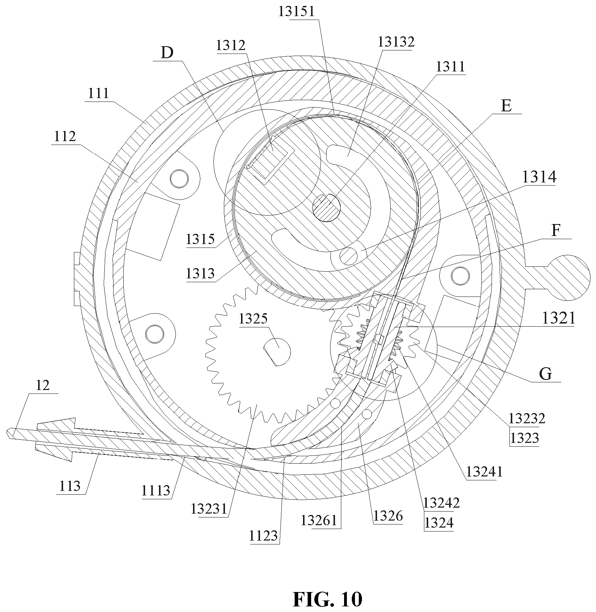

The pulling and twisting of the drive wire 121 of the actuator unit 12 is also realized based on the driving force output from the instrument driving device 20. Specifically, the driving force is transmitted through a push-pull transmission assembly 131 and a rotation transmission assembly 132 of the transmission unit 13. Referring to FIGS. 9 and 10 together, FIG. 9 is a schematic view of the overall structure of the transmission unit described according to the embodiment, and FIG. 10 is a cross-sectional view taken along line C-C of FIG. 8.

As shown in FIG. 9, the transmission unit 13 includes the push-pull transmission assembly 131 and the rotation transmission assembly 132 which are both arranged on the transmission base plate 133.

The push-pull transmission assembly 131 includes a first terminal 1312, a fixing disc 1313 and a first driving shaft 1311.

The fixing disc 1313 is coaxially and fixedly connected with the first driving shaft 1311, and the first driving shaft 1311 is inserted into the transmission base plate 133 such as to rotate under the driving force of the instrument driving device 20 and drive the fixing disc 1313 to swing around the rotation center of the fixing disc 1313.

The first terminal 1312 is fixed to the fixing disc 1313 and can move with the fixing disc 1313 when it is rotated. The proximal end of the drive wire 121 is connected to the first terminal 1312, such that the drive wire 121 can be driven by the first terminal 1312 to be pushed out or retracted along a predetermined trajectory and can rotate relative to the first terminal 1312. It can be understood that the swing range of the fixing disc 1313 needs to meet requirements of the pulling stroke of the first terminal 1312, that is, the pushing-out or retracting displacement required by the actuator 122.

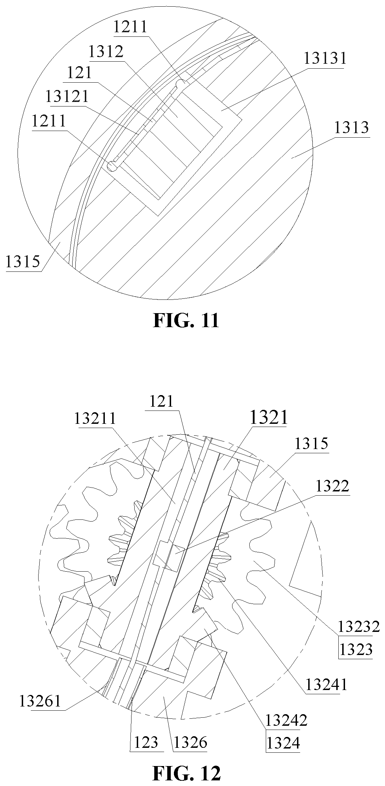

Referring to FIGS. 10 and 11 together, FIG. 11 is an enlarged view of part D in FIG. 10.

The first terminal 1312 is embedded in the fixing disc 1313. Specifically, an outer circumferential surface of the fixing disc 1313 is provided with a mounting groove 13131 fitted with the first terminal 1312. In other implementations, the entire first terminal may be arranged inside the fixing disc, rather than being limited to being embedded in the outer circumferential surface of the fixing disc 1313 (not shown).

In the embodiment, the drive wire 121 passes through a first through hole 13121 of the first terminal 1312, and a main body of the drive wire 121 is provided with two limit bumps 1211 which are respectively located at two end of the first through hole 13121. The size of the limit bump 1211 is larger than that of the first through hole 13121. When the fixing disc 1313 rotates forward and backward, the drive wire 121 can be pushed out and retracted under the limit between the first terminal 1312 and the limit bump 1211 on the corresponding side, so as to meet the operation requirements during surgery.

In addition, there is a radial gap between the drive wire 121 and the first through hole 13121 of the first terminal 1312, that is, the drive wire 121 has rotational degree of freedom relative to the first terminal. When the drive wire is rotated under the drive of the rotation transmission assembly 132, the drive wire can be rotated relative to the first through hole 13121 without interference with the first terminal. In other implementations, the first terminal and the fixing disc may be of an integrated structure, that is, the functional structure of the first terminal can be integrated into the fixing disc. Comparatively speaking, the first terminal and the fixing disc adopting a split structure as shown in the figures have good processing performance.

In order to locate bidirectional limit positions of the stretching movement, a rotation limit pair may be provided between the fixing disc 1313 and the transmission base plate 133. As shown in FIGS. 9 and 10, the body of the fixing disc 1313 is provided with an arc groove 13132, and correspondingly, the transmission base plate 133 is provided with a limit block 1314 arranged in the arc groove 13132 of the fixing disc 1313. A circle center of the arc groove 13132 is concentric with the rotation center of the fixing disc 1313. In this way, when the fixing disc 1313 is driven by the first driving shaft 1311 to rotate forward and backward, two side ends of the arc groove 13132 in the circumferential direction can abut against the limit block 1314. That is to say, the limit working positions of the push-pull transmission assembly 131 are determined by the arc groove 13132 and the limit block 1314, which can prevent the rotation angle of the fixing disc 1313 from being excessive to affect the actuation performance, and meet the requirements of the pulling stroke of the first terminal 1312.

In the embodiment, two ends of the limit block 1314 for abutting are in convex arc shape to be matched with the structural form of the two side ends of the arc groove 13132.

A first restraint member 1315 is fixedly provided on the transmission base plate 133. The first restraint member 1315 includes a guide body E and a holding body F that are provided with constraint cavities 13151. The drive wire 121 is arranged in the constraint cavities 13151. The guide body E is cylindrical, and the fixing disc 1313 is arranged inside the guide body E. The holding body F and the guide body E are fixedly connected to each other, or may be of an integrated structure. In this way, under the drive of the first terminal 1312, the drive wire 121 can be pushed out or retracted on the predetermined trajectory constructed by the constraint cavities 13151.

As shown in FIG. 10, the constraint cavity 13151 on an inner wall of the guiding body E is an open cavity to form a guiding section, and the constraint cavity 13151 in the holding body F is a closed cavity to form a holding section. In this way, the rotation guidance of the fixing disc 1313 can be established by the arc-shaped inner wall, and the predetermined trajectory for guiding the drive wire 121 can be established by the open cavity of the guiding section and the closed cavity of the holding section.

As shown in FIGS. 9 and 10 again, in the embodiment, the rotation transmission assembly 132 includes a rotation shaft 1321, a second terminal 1322, a spur gear set 1323, a bevel gear set 1324 and a second driving shaft 1325.

A driving gear of the bevel gear set 1324 is connected to the second driving shaft 1325. The second driving shaft 1325 is inserted into the transmission base plate 133 to be rotated under the driving force of the instrument driving device 20, and drives the rotation shaft 1321 to rotate around its rotation center through the sequential transmission of the spur gear set 1323 and the bevel gear set 1324.

In the embodiment, on the rotational transmission path, the spur gear set 1323 is a primary gear set, and a driving gear 13231 of the spur gear set 1323 is coaxially fixed to the second driving shaft 1325. The bevel gear set 1324 is a secondary gear set, and a driving bevel gear 13241 of the bevel gear set 1324 is coaxially fixed to a driven gear 13232 of the spur gear set 1323, and a driven bevel gear 13242 of the bevel gear set 1324 is coaxially fixed to the rotation shaft 1321.

It should be noted that the push-pull transmission assembly 131 and the rotation transmission assembly 132 can be reasonably arranged on the transmission base plate 133 by determining a distance between the axes of the driving and driven gears of the spur gear set 1323. Moreover, the speed ratio of the two-stage transmission mechanism can be further adjusted to meet the design requirements of different products, which has good adaptability.

The second terminal 1322 is arranged on the rotation shaft 1321, and the drive wire 121 is fixed by the second terminal 1322 and can be rotated with the rotation shaft 1321 when it is rotated. The second terminal 1322 can be rotated under drive of the rotation shaft 1321, and the second terminal 1322 can slide relative to the rotation shaft 1321 in the pulling direction of the drive wire 121. The sliding stroke of the second terminal 1322 also needs to meet the requirements of the pulling stroke of the first terminal 1312.

Referring to FIGS. 10 and 12 together, FIG. 12 is an enlarged view of part G in FIG. 10.

The second terminal 1322 is embedded in the rotation shaft 1321. Specifically, the middle of the rotation shaft 1321 is provided with a mounting hole 13211, and the drive wire 121 can extend into the constraint cavity 13151 of the first constraint member through the mounting hole 13211. In the embodiment, the second terminal 1322 fixedly connected to the drive wire 121 is inserted into the mounting hole 13211, and the second terminal 1322 and the mounting hole 13211 have rectangular cross sections matched with each other. In this way, when the rotation shaft 1321 rotates, the second terminal 1322 can rotate synchronously to drive the drive wire 121 to twist. Moreover, the second terminal 1322 has sliding degree of freedom relative to the rotation shaft 1321, that is, the second terminal 1322 can move axially relative to the rotation shaft 1321. When pulled by the push-pull transmission assembly 131, the drive wire can rotate relative to the mounting hole 13211 without interference with the rotation shaft.

In other implementations, the cross sections of the second terminal 1322 and the mounting hole 13211 may be of other structures, such as, but not limited to, having other polygon shapes or having a flat surface for a circumferential limit, as long as the functional requirements that the second terminal can slide in the mounting hole and can rotate synchronously with the rotation shaft can be met, which is within the protection scope of the present application.

A second constraint member 1326 is fixedly provided on the transmission base plate 133. As shown in FIGS. 9 and 10, in the axial direction of the rotation shaft 1321, one end of the second constraint member 1326 faces one end of the holding body F of the first constraint member 1315, and the second constraint member 1326 and the first constraint member 1315 respectively support both ends of the shaft, so that the two ends of the rotation shaft 1321 can be reliably pivoted to meet the functional requirement of relative rotation.

The second constraint member 1326 is provided with a second through hole 13261. Correspondingly, the instrument storage device 112 of the instrument delivery unit 11 is provided with a pass-through port 1123, and the pass-through port 1123 is obliquely arranged, so that the flexible body of the actuator unit 12 extends and transitions to the helical accommodating groove 11211 on the outer surface of the instrument storage device 112. The size of the second through hole 13261 may be substantially the same as the size of the sleeve 123 of the flexible body of the actuator unit 12 to reliably fix an end of the sleeve 123.

In general, when the instrument needs to be pushed or pulled and rotated, it is driven by the push-pull transmission assembly and the rotation transmission assembly individually. The first terminal of the push-pull transmission assembly only constrains the axial position of the drive wire, and the rotation shaft of the rotation transmission assembly 132 only constrains the circumferential position of the second terminal. The two motions can be effectively decoupled, and can be constrained in two degrees of freedom.

Furthermore, the flexible instrument 10 according to the embodiment further includes a connecting unit 14 for connection with external devices, such as, but not limited to, a power source and a signal source and a water line. Referring to FIGS. 1, 4 and 13, FIG. 13 is a schematic view showing assembly of the connecting unit according to the embodiment.

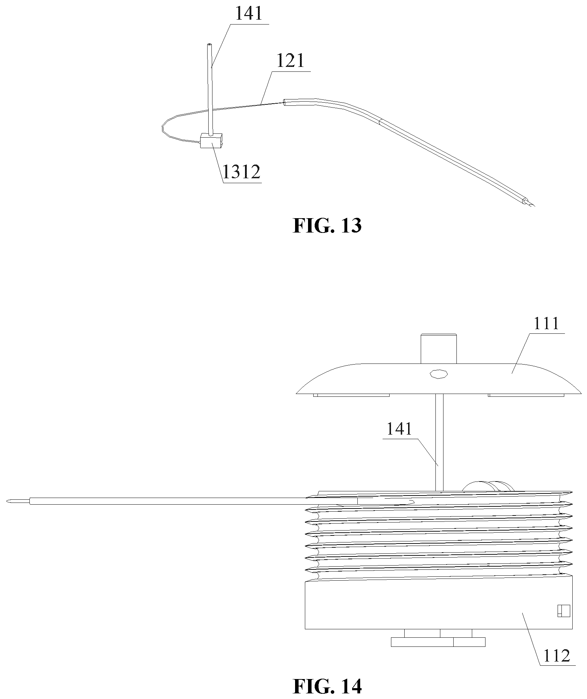

The connecting unit 14 may include a two-way connector 141 with a through inner cavity. One end of the two-way connector 141 may extend out of a top end of the housing 111, and the other end of the two-way connector 141 is inserted into the first terminal 1312 and connected to the drive wire 121 of the actuator unit 12, thereby realizing the connection with power supply and signal source and the connection with water line. Referring to FIG. 14, it shows a schematic view showing assembly of the two-way connector. In order to clearly illustrate the connection orientation of the two-way connector, part of the housing is not shown in the figure.

In an implementation, in a use state where a water line connection needs to be established, for example, but not limited to the case where the actuator unit 12 is an injection needle as shown in the figure, an external liquid tube (not shown) may be communicated with the hollow drive wire 121 of the actuator unit 12 through the two-way connector 141, so as to supply injection liquid to the distal end.

It should be understood that, in the first terminal 1312, a swivel joint may be provided between the drive wire 121 and an insertion end of the two-way connector 141 to meet the requirements of an application scenario where the drive wire 121 can rotate relative to the first terminal 1312. In an application scenario where an injection needle is used and the drive wire does not need to rotate, there is no need to provide a swivel joint between the drive wire 121 in the first terminal 1312 and the two-way connector 141, that is, the drive wire 121 and the two-way connector 141 are directly communicated with each other.

In other implementations, in a use state where power supply or communication needs to be established, an external cable passes through the two-way connector 141 made of metal material and is connected to the metal drive wire 121 to meet the corresponding functional requirements.

In order to facilitate the rapid assembly of the whole machine, in the embodiment, a detachable connection mechanism is provided between the flexible instrument 10 and the instrument driving device 20, and is specifically arranged between the instrument driving device 20 and the instrument storage device 112 as well as the transmission unit 13, so as to realize the rapid assembly operation and meet the functional requirements of transmitting the driving force.

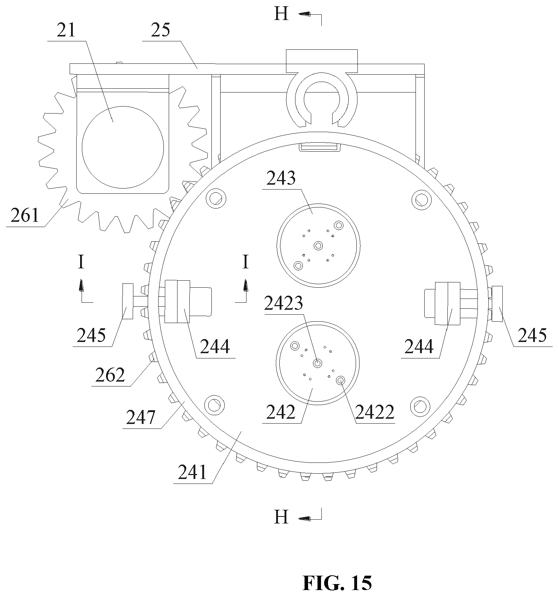

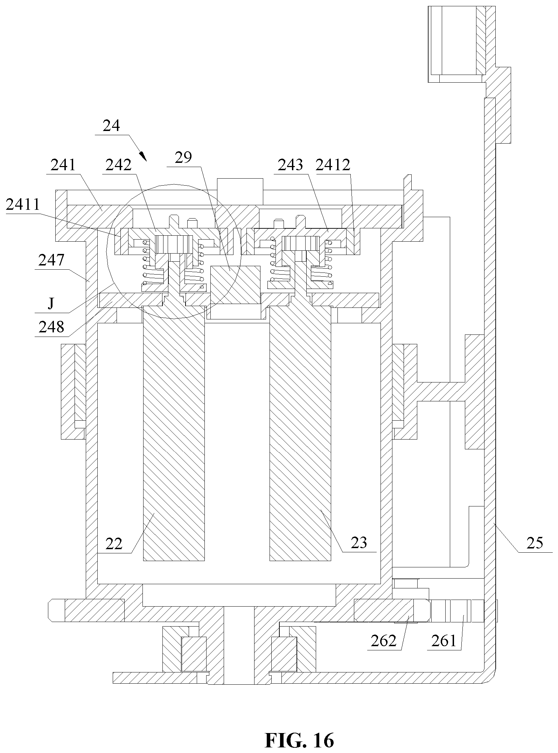

Referring to FIGS. 15 and 16, FIG. 15 is a top view of the instrument driving device 20 according to the embodiment, and FIG. 16 is a cross-sectional view taken along line H-H in FIG. 15.

As shown, a connecting part 24 for outputting power is provided at a top of the instrument driving device 20, and includes a driving base plate 241, a first driving transmission disc 242, a second driving transmission disc 243 and a casing 247. The driving base plate 241 is fixedly arranged at a top of the casing 247. The driving base plate 241, as an interface connector for outputting a driving force of a first driving part 21, is configured to transmit power to the instrument storage device 112. The first driving transmission disc 242, as an interface connector for outputting a driving force of a second driving part 22, is configured to transmit power to the first driving shaft 1311 for pulling the drive wire. The second driving transmission disc 243, as an interface connector for outputting a driving force of a third driving part 23, is configured to transmit power to the second driving shaft 1325 for twisting the drive wire.

A mounting base plate 248 is fixedly arranged in the casing 247, and the second driving part 22 and the third driving part 23 are fixed to the mounting base plate 248. The mounting base plate 248, as a basic mounting structure, may be of any other structure.

It can be understood that an execution drive connector on the instrument side may be the first and second driving shafts, or first and second driven transmission discs connected to the corresponding driving shafts as described below. In an implementation, the execution drive connector is determined based on overall design requirements of the product. From the perspective of driving force transmission, any execution drive connector that can realize reliable power transmission is within the protection scope of the present application.

Referring to FIGS. 1 and 15 together, in the embodiment, the first driving part 21 is provided on a fixing support 25 and transmits power to the casing 247 through a conveying gear set 26. An input gear 261 of the conveying gear set 26 is coaxially fixed to an output end of the first driving part 21, and an output gear 262 of the conveying gear set 26 is fixedly connected to a bottom of the casing 247, thereby transmitting power to the driving base plate 241 fixed to the casing 247.

In other implementations, the first driving part 21 may adopt a belt transmission mechanism to transmit power to the casing 247. Compared with an implementation that both rotational and axial driving forces are provided on the driving side, the solution according to the embodiment has the characteristics of simple structure and can reduce the manufacturing cost and maintenance cost of the driving mechanism.

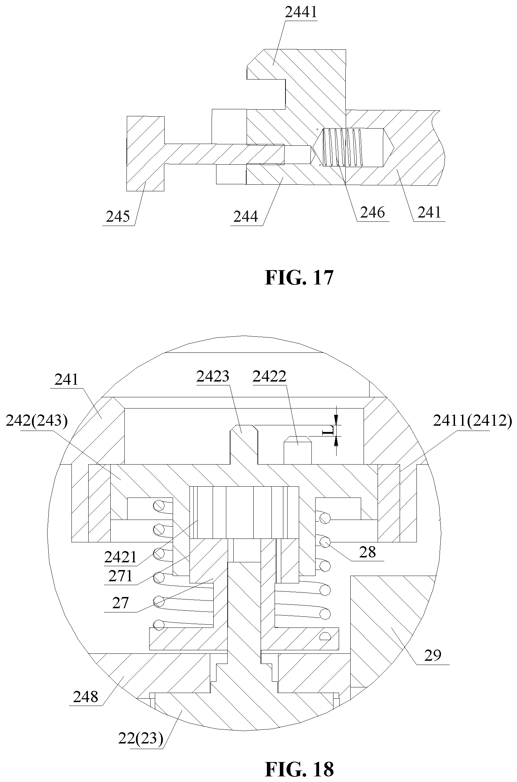

In the embodiment, the driving base plate 241 faces the transmission base plate 133. A snap fastener 244 is provided on the driving base plate 241, and the transmission base plate 133 is correspondingly provided with an engagement groove 1331, as shown in FIGS. 3 and 17, wherein FIG. 17 is a partial sectional view taken along line I-I in FIG. 15, showing assembly between the driving base plate and the snap fastener.

After assembly, the snap fastener 244 is placed in the engagement groove 1331 to form a circumferential rotation limit pair. When the first driving part 21 drives the driving base plate 241 to rotate, the transmission base plate 133 can be driven to rotate synchronously by the circumferential rotation limit pair, and thus the instrument storage device 112 fixed to the transmission base plate 133 can be driven to rotate, thereby transporting the actuator unit 12.

It can be understood that the engagement groove 1331 engaged with the snap fastener 244 to establish the circumferential rotation limit pair is not limited to being arranged in the transmission base plate. In other implementations, the engagement groove may be provided in the instrument storage device, or the engagement groove may be formed on both the transmission base plate and the instrument storage device.

In the embodiment, two groups of the matched snap fasteners 244 and the engagement grooves 1331 are provided, and are symmetrically arranged, so that the stress is relatively balanced. It can be understood that, in other implementations, the number of groups of the matched snap fasteners 244 and the engagement grooves 1331 may be more than two, which are arranged at intervals in the circumferential direction.

Further, the snap fastener 244 can move radially relative to the driving base plate 241, that is, the snap fastener 244 can slide in the engagement groove1331. The snap fastener 244 has a hook portion 2441 extending outward from the body of the snap fastener. Correspondingly, a side wall of the instrument storage device 112 is provided with an engagement opening 1124 engaged with the hook portion 2441. In this way, when the snap fastener 244 is in an extended working position, the hook portion 2441 can be inserted into the engagement opening 1124 to prevent the separation of the instrument storage device 112, so as to realize the conveying drive and the axial locking.

In order to improve the operability, a button 245 is provided at an outer side of the snap fastener 244, and a snap fastener return spring 246 is provided at an inner side of the snap fastener 244. As shown in FIGS. 15 and 17, the snap fastener return spring 246 can be pre-compressed between the snap fastener 244 and the driving base plate 241, so that the snap fastener 244 can be reliably maintained in the extended working position.

During disassembly, an operator applies a force to the button 245, so that the snap fastener 244 slides inward along the engagement groove 1331, the snap fastener return spring 246 being further deformed, until the hook portion 2441 is disengaged from the engagement opening 1124. Then, the flexible instrument 10 can be detached. It can be understood that, in other implementations, the return spring may be of any other structural form, such as, but not limited to, a return member made of rubber materials or the like, or a return member with an elastic sheet structure.

Further, in order to avoid the possible influence caused by the exposure of the snap-fit structure, in the embodiment, the casing 247 extends axially upward to the outside of the driving base plate 241 and the snap fastener 244 on the driving base plate 241, and is provided with two pass-through holes 2471 which radially correspond to the two buttons 245, so that push rods of the buttons 245 are fixedly connected to bodies of the snap fasteners 244 through the pass-through holes 2471, respectively.

In the embodiment, the driving base plate 241 is provided with a first step hole 2411 and a second step hole 2412, and each step hole has a small-diameter upper section and a large-diameter lower section to form a step. The first driving transmission disc 242 is rotatably arranged in the large-diameter section of the first step hole 2411, and the second driving transmission disc 243 is rotatably arranged in the large-diameter section of the second step hole 2412. The first driving transmission disc 242 and the second driving transmission disc 243 can be driven to rotate by corresponding driving parts and can move axially in the corresponding large-diameter sections.

The way in which the first driving transmission disc 242 is assembled with the corresponding driving part is the same as the way in which the second driving transmission disc 243 is assembled with the corresponding driving part, and the first driving transmission disc 242, as an example, will be described in detail below.

Referring to FIGS. 16 and 18 together, FIG. 18 is an enlarged view of part J in FIG. 16.

A surface of the first driving transmission disc 242 facing the second driving part 22 is provided with a key groove 2421. An output end of the second driving part 22 is provided with a sliding shaft sleeve 27. One end of the sliding shaft sleeve 27 facing the first driving transmission disc 242 is provided with an outer sliding key 271 which can be inserted into the key groove 2421 of the first driving transmission disc 242. The key groove 2421 and the outer sliding key 271 can rotate coaxially and slide axially relative to each other. That is to say, the key groove 2421 and the outer sliding key 271 constitute a rotation matched pair and an axial sliding matched pair.

It can be understood that the key groove 2421 and the outer sliding key 271 that are engaged with each other may be of different cross-sectional shapes, such as, but not limited to, a rectangle, a hexagon or other polygons, or may be of a standard spline structure, as long as they can transmit rotational torque and slide axially relative to each other.

In an implementation, the key groove 2421 and the outer sliding key 271 that are matched with each other may also be configured in reverse. In other words, the key groove may be provided on the sliding shaft sleeve, and the outer sliding key may be correspondingly provided on the first driving transmission disc (not shown), which can also meet the functional requirements that the key groove and the outer sliding key rotate coaxially and slide axially relative to each other.

As shown in FIGS. 16 and 18, a transmission disc return spring 28 is provided between the first driving transmission disc 242 and the sliding shaft sleeve 27. Under normal conditions, the transmission disc return spring 28 is pre-compressed between the first driving transmission disc 242 and the sliding shaft sleeve 27. Under the action of the transmission disc return spring 28, the first driving transmission disc 242 can axially abut against the step of and the first step hole 2411.

Moreover, a surface of the first driving transmission disc 242 facing the first driven transmission disc 134 is provided with a connecting post 2422 extending in the axial direction, and the connecting post 2422 is configured to be fitted with a connecting hole 1341 provided in the axial direction in the first driven transmission disc 134. The connecting post 2422 and the connecting hole 1341 are eccentrically arranged with respect to rotation centers of the driving and driven transmission discs to transmit torque and drive the driven transmission disc to rotate synchronously.

In the embodiment, a bottom of the transmission base plate 133 is provided with the first driven transmission disc 134 and the second driven transmission disc 135 which are respectively arranged on the push-pull transmission path and the rotation transmission path of the actuator. Specifically, the first driven transmission disc 134 is fixedly connected to a shaft end of the first driving shaft 1311, and is connected to the first driving transmission disc 242, so as to perform push-pull transmission. The second driven transmission disc 135 is fixedly connected to a shaft end of the second driving shaft 1325, and is connected to the second driving transmission disc 243, so as to perform rotation transmission.

The way in which the first driven transmission disc 134 is assembled with the corresponding driving transmission disc is the same as the way in which the second driven transmission disc 135 is assembled with the corresponding driving transmission disc, and the first driven transmission disc 134, as an example, will be described in detail below.

As shown in FIG. 7, the first driven transmission disc 134 is provided with two connecting holes 1341 to balance load during power transmission, so as to improve transmission stability. In other implementations, the number of connecting holes 1341 may be determined based on the overall design of the product.

In addition, a guide hole 1342 may be provided at the rotation center of the first driven transmission disc 134, and correspondingly, a guide post 2423 is provided at the rotation center of the first driving transmission disc 242. In the axial direction, an outer extension end of the guide post 2423 exceeds an outer extension end of the connecting post 2422, as shown by a dimension mark L in FIG. 18. In this way, during assembly, insertion of the guide post 2423 may precede insertion of the connecting post 2422, which facilitates quick connection between the first driven transmission disc 134 and the first driving transmission disc 242.

In an implementation, the connecting hole 1341 and the connecting post 2422 that are matched with each other, and the guide hole 1342 and the guide post 2423 that are matched with each other, may also be configured in reverse. That is to say, the connecting hole and the guide hole are arranged on the first driving transmission disc, and the connecting post and the guide post are arranged on the first driven transmission disc, which can also achieve the functions of transmitting torque and connecting guidance.

In the actual assembly operation, the flexible instrument 10 is inserted into or connected to the connecting part 24 of the instrument driving device 20 by the transmission unit 13. The connecting hole 1341 and the guide hole 1342 of the first driven transmission disc 134 are respectively aligned with the connecting post 2422 and the guide post 2423 of the first driving transmission disc 242, and the connecting post 2422 and the guide post 2423 are respectively pressed into the connecting hole 1341 and the guide hole 1342. When pressed by an operator, the first driven transmission disc 134 can press the first driving transmission disc 242 to move downwards synchronously due to the axial sliding matched pair, and the transmission disc return spring 28 is deformed under pressure to provide buffering adaptability, which can avoid the rigid interference influence during insertion of the connecting post 2422 and the guide post 2423. After the connection is completed, the transmission disc return spring 28 releases elastic deformation energy and pushes the first driven transmission disc 134 to move axially to abut against the step of the first step hole 2411.

In addition, in order to monitor the installation state in real time, in the embodiment, a detection element 29 is further provided. The detection element 29 may be a pressure-sensitive detection element located on the axial movement track of the first driving transmission disc 242 and the second driving transmission disc 243. When the flexible instrument is mounted to the driving device, the first driving transmission disc 242 and the second driving transmission disc 243 are pressed to move downwards synchronously, so as to press the pressure-sensitive detection element to generate a signal. When connected in place, the first driving transmission disc 242 and the second driving transmission disc 243 are moved upward and press against the steps of the corresponding step holes respectively under the action of the corresponding transmission disc return springs 28. The signal disappears after the pressure is removed from the pressure-sensitive detection element, thereby detecting the installation state. Therefore, the operator can realize blind connection between the instrument side and the driving side, which can further improve the operability.

It should be noted that the detection element may be in any other device form, which may be determined based on the actual product design requirements and is not limited to the device types and the configuration or position shown in the figures.

As described above, the action mechanism of the drive transmission side is the same as that of the push-pull transmission side. It can be understood that the functional implementation of the drive transmission side is the same as that of the push-pull transmission side, which is not repeated here.

In the aforementioned embodiment, the transmission connection is established by the first transmission thread 1111 of the housing 111 and the second transmission thread 11212 of the instrument storage device 112. In other words, the first transmission thread 1111 is an internal thread and the second transmission thread 11212 is an external thread. When the instrument storage device 112 rotates to convey the flexible body of the actuator unit 12, the housing 111 cooperates to move axially. In other implementations, the internal and external transmission threads engaged with each other may also be configured in reverse.

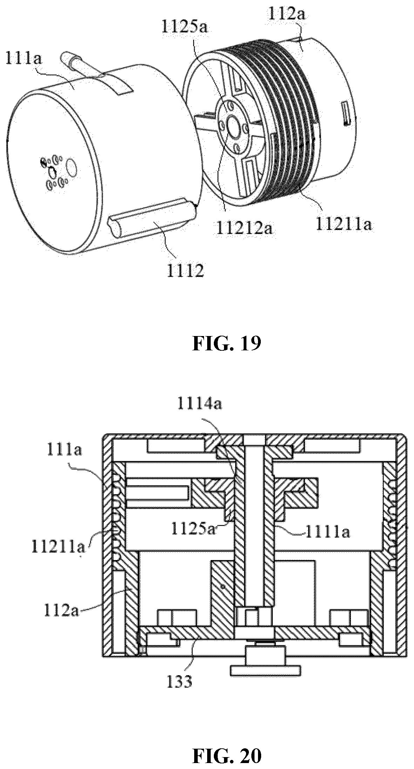

Referring to FIGS. 19 and 20 together, FIG. 19 shows a schematic view showing assembly of a flexible instrument according to another embodiment, and FIG. 20 is an axial cross-sectional view of the flexible instrument shown in FIG. 19. In order to clearly illustrate the difference and relation between this embodiment and the previous embodiment, the components or structures performing the same function are illustrated in the figures with the same reference signs.

This embodiment is different from the previous embodiment in that: a lead screw 1114a is provided in the middle of the housing 111a, and a first transmission thread 1111a is provided on the lead screw 1114a; and correspondingly, a nut 1125a is provided in the instrument storage device 112a, and the nut 1125a is provided with a second transmission thread 11212a, and is engaged with the lead screw 1114a.

The nut 1125a is fixedly arranged on an inner wall of the instrument storage device 112a. When the instrument storage device 112a rotates for transportation, the nut 1125a rotates synchronously. The housing 111a moves axially synchronously based on the limiting and guiding function of the limiting guide portion 1112 as well as the lead screw and the nut that are engaged with each other.

As shown in FIG. 20, an outer circumferential surface of the instrument storage device 112a is only provided with a helical accommodating groove 11211a for winding and accommodating the flexible body. The housing 111a has a smooth inner wall which can form a radial constraint on the flexible body.

Other structures and connection relationships are the same as those in the previous embodiment, so they are not repeated here.

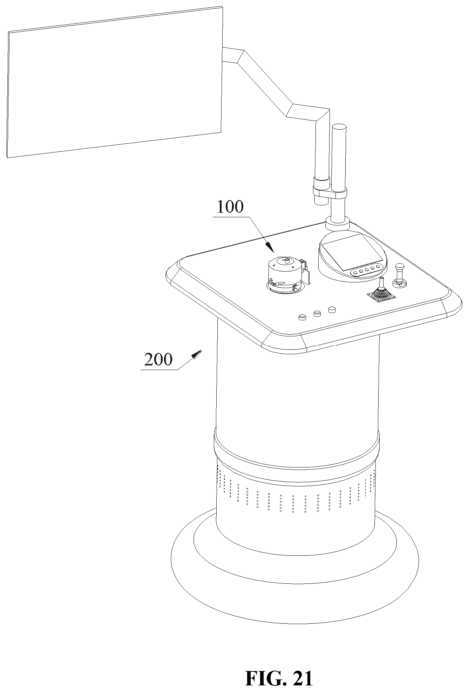

The flexible surgical instrument according to the embodiment may be applied to an Endoscopic Nurse/Technician Robot. As shown in FIG. 21, the flexible surgical instrument 100 is mounted on a robot body 200 to assist an endoscopist in operating a surgical instrument, reasonably realizing the effective storage of the body of the actuator unit and avoiding cross contamination, thereby effectively improving the doctor's work efficiency.

Ordinal numbers "first" and "second" herein are only used to describe the construction or structure of the same function in the technical solutions. It can be understood that the use of ordinal numbers "first" and "second" does not constitute an understanding limitation of the technical solutions according to the present application.

The above is only the preferred embodiments of the present application. It should be pointed out that, several improvements and modifications can be made by those skilled in the art without departing from the principle of the present application, and these improvements and modifications should also be regarded as falling within the protection scope of the present application.

## Claims

1. An instrument delivery unit for delivering and storing an actuator unit having a flexible body, comprising:
a housing comprising: an internal accommodation space; an instrument outlet provided in a side wall of the housing; a first transmission thread provided on the housing; and a limiting guide portion provided on an outer side of the housing to be engaged with a fixed structure and configured to guide an axial movement of the housing and prevent a circumferential rotation of the housing; and
an instrument storage device, at least part of which is arranged in the housing, wherein an outer circumferential surface of the instrument storage device is provided with a helical accommodating groove for winding and storing the flexible body of the actuator unit, the instrument storage device is provided with a second transmission thread which is in transmission connection with the first transmission thread, and the housing is configured to move axially synchronously when the instrument storage device rotates so that the flexible body of the actuator unit remains aligned with the instrument outlet in two dimensions.

2. The instrument delivery unit according to claim 1, wherein
the first transmission thread is arranged on an inner wall surface of the housing, and the outer circumferential surface of the instrument storage device is provided with a helical groove which comprises an inner groove section and an outer groove section formed in sequence in a radial direction; a wall of the inner groove section defines the helical accommodating groove, and a wall of the outer groove section forms the second transmission thread.

3. The instrument delivery unit according to claim 1, wherein
a lead screw is provided in the middle of the housing, and the first transmission thread is arranged on the lead screw; a nut is fixedly provided in the instrument storage device, and the nut is provided with the second transmission thread.

4. The instrument delivery unit according to any one of claims 1 to 3, wherein the housing is cylindrical and has one open end, one end of the instrument storage device is arranged in the housing, and the other end of the instrument storage device is provided with a delivery drive connector.

5. A flexible instrument, comprising:
the instrument delivery unit according to any one of claims 1 to 4;
an actuator unit comprising an actuator and a flexible body; wherein the flexible body comprises a drive wire and a sleeve in which the drive wire is arranged, and the actuator is arranged at a distal end of the drive wire; and
a transmission unit connected to the instrument storage device of the instrument delivery unit and configured to drive the actuator to move via the drive wire of the actuator unit; wherein
the transmission unit is provided with an execution drive connector for being fitted with an instrument driving device;
the transmission unit comprises a transmission base plate fixedly connected to the instrument storage device, and a push-pull transmission assembly and a rotation transmission assembly that are arranged on the transmission base plate; and
the push-pull transmission assembly is configured to push out or retract the drive wire, and the rotation transmission assembly is configured to twist the drive wire.

6. The flexible instrument according to claim 5, wherein
the push-pull transmission assembly comprises a fixing disc and a first driving shaft, and the first driving shaft is inserted into and arranged on the transmission base plate and is configured to be in transmission connection with the instrument driving device through the execution drive connector; the fixing disc is coaxially and fixedly connected to the first driving shaft, and a proximal end of the drive wire is connected to the fixing disc, and the drive wire is configured to be pushed out or retracted along a predetermined trajectory under driving of the fixing disc, and the drive wire has a rotational degree of freedom relative to the fixing disc.

7. The flexible instrument according to claim 6, wherein
a first terminal is embedded in an outer circumferential surface of the fixing disc, and the first terminal has a first through hole; the drive wire passes through the first through hole of the first terminal, and a body of the drive wire is provided with two limit bumps which are respectively located at two ends of the first through hole of the first terminal; a radial gap is provided between the drive wire and the first terminal at the first through hole, and a size of each of the two limit bumps is larger than that of the first through hole.

8. The flexible instrument according to claim 7, wherein a rotation limit pair is provided between the fixing disc and the transmission base plate, and the rotation limit pair is configured to limit a rotation angle of the fixing disc.

9. The flexible instrument according to claim 8, wherein
a body of the fixing disc is provided with an arc groove, and a circle center of the arc groove is concentric with a rotation center of the fixing disc; the transmission base plate is provided with a limit block, the limit block is arranged in the arc groove, and two side ends of the arc groove are configured to abut against the limit block to limit the rotation angle of the fixing disc.

10. The flexible instrument according to claim 9, wherein
a first constraint member is fixedly arranged on the transmission base plate, and the first constraint member comprises a cylindrical guide body and a holding body, and the fixing disc is arranged in the guide body;
each of the guide body and the holding body is provided with a constraint cavity for accommodating the drive wire, so that the drive wire is pushed out or retracted along the predetermined trajectory; and
the constraint cavity on an inner wall of the guide body is an open cavity, and the constraint cavity of the holding body is a closed cavity.

11. The flexible instrument according to claim 10, wherein
the rotation transmission assembly comprises a rotation shaft, a second terminal, a bevel gear set and a second driving shaft; the second driving shaft is inserted into and arranged on the transmission base plate, and is configured to be in transmission connection with the instrument driving device through the execution drive connector; a driving gear of the bevel gear set is connected to the second driving shaft, and the rotation shaft is connected to a driven wheel of the bevel gear set; and the drive wire is fixed to the second terminal, the second terminal is arranged on the rotation shaft and is configured to rotate under driving of the rotation shaft, and the second terminal has a degree of freedom of sliding in a pulling direction of the drive wire relative to the rotation shaft.

12. The flexible instrument according to claim 11, wherein the second terminal is embedded in a mounting hole in the middle of the rotation shaft, the second terminal and the mounting hole have rectangular cross sections matched with each other, and the drive wire is configured to extend into the constraint cavities of the first constraint member through the mounting hole.

13. The flexible instrument according to claim 12, wherein a second constraint member is fixedly provided on the transmission base plate, one end of the second constraint member faces one end of the holding section of the first constraint member in an axial direction of the rotation shaft, and two opposite ends of the rotation shaft are respectively pivoted with the second constraint member and the first constraint member.

14. The flexible instrument according to claim 13, wherein
the second constraint member is provided with a second through hole, and a size of the second through hole of the second constraint member is adapted to a size of the sleeve of the actuator unit to fix an end of the sleeve; and the instrument storage device is provided with a pass-through port, and the flexible body of the actuator unit extends and transitions into a helical accommodating groove on an outer surface of the instrument storage device through the pass-through port of the instrument storage device.

15. A flexible surgical instrument, comprising the flexible instrument according to any one of claims 5 to 14 and an instrument driving device for outputting a driving force to the flexible instrument.

16. The flexible surgical instrument according to claim 15, wherein the instrument driving device comprises:
a first driving part including an output end for outputting a rotational driving force;
a casing in transmission connection with the output end of the first driving part;
a driving base plate fixedly arranged on the casing and configured to transmit the rotational driving force to the flexible instrument; and
a second driving part and a third driving part fixedly arranged on the casing by a mounting base plate; wherein each of the second driving part and the third driving part comprises an output end for outputting a rotational driving force, and the output ends are configured to transmit the rotational driving forces to the push-pull transmission assembly and the rotation transmission assembly of a transmission unit, respectively.

17. The flexible surgical instrument according to claim 16, wherein
the instrument driving device further comprises a first driving transmission disc and a second driving transmission disc, the first driving transmission disc is in transmission connection with the output end of the second driving part, and the second driving transmission disc is in transmission connection with the output end of the third driving part; the transmission unit further comprises a first driven transmission disc connected to the push-pull transmission assembly and a second driven transmission disc connected to the rotation transmission assembly; and the first driving transmission disc is in transmission connection with the first driven transmission disc, and the second driving transmission disc is in transmission connection with the second driven transmission disc.

18. The flexible surgical instrument according to claim 17, wherein
one of the first driving transmission disc and the first driven transmission disc is provided with a connecting hole extending in an axial direction, the other of the first driving transmission disc and the first driven transmission disc is provided with a connecting post extending in the axial direction, and the connecting hole and the connecting post that are matched with each other are arranged eccentrically with respect to rotation centers of the first driving transmission disc and the first driven transmission disc; and
one of the second driving transmission disc and the second driven transmission disc is provided with a connecting hole extending in the axial direction, the other of the second driving transmission disc and the second driven transmission disc is provided with a connecting post extending in the axial direction, and the connecting hole and the connecting post that are matched with each other are arranged eccentrically with respect to rotation centers of the second driving transmission disc and the second driven transmission disc.

19. The flexible surgical instrument according to claim 18, wherein
the driving base plate is provided with a first step hole and a second step hole, and each of the first step hole and the second step hole includes a small-diameter section and a large-diameter section which are sequentially arranged from top to bottom to form a step; and
the first driving transmission disc is rotatably arranged in the large-diameter section of the first step hole, and the second driving transmission disc is rotatably arranged in the large-diameter section of the second step hole.

20. The flexible surgical instrument according to claim 19, wherein
a surface of each of the first driving transmission disc and the second driving transmission disc facing the corresponding driving part is provided with a key groove;
the output end of each of the second driving part and the third driving part is provided with a sliding shaft sleeve, and one end of the sliding shaft sleeve facing the corresponding driving transmission disc is provided with an outer sliding key;
the outer sliding key is inserted into the corresponding key groove, and the outer sliding key and the corresponding key groove are configured to rotate coaxially and slide axially relative to each other; and
a transmission disc return spring is provided between each of the first driving transmission disc and the second driving transmission disc, and the corresponding sliding shaft sleeve; and the transmission disc return spring is configured to be pre-compressed between the sliding shaft sleeve and the corresponding driving transmission disc under a normal condition.

21. The flexible surgical instrument according to claim 20, wherein a detection element is provided on the mounting base plate, and the detection element is located on an axial movement trajectory of the first driving transmission disc and the second driving transmission disc.
